# EUROPEAN PATENT APPLICATION

(11) **EP 4 227 418 A1**
(43) Date of publication of application: **16.08.2023**
(21) Application number: 23155901.4
(22) Date of filing: 09.02.2023
(51) Int. Cl.: C12P 19/02, B01D 45/00, C12P 19/14

(54) **CELLULOSE SACCHARIFICATION METHOD**

(30) Priority: 10.02.2022 JP 2022019357
(71) Applicant: Seiko Epson Corporation, Tokyo 160-8801 (JP)
(72) Inventor: TANAKA, Hideki, Suwa-shi, 392-8502 (JP); NAKAI, Yoko, Suwa-shi, 392-8502 (JP); NAKASHIMA, Yoshiki, Suwa-shi, 392-8502 (JP); YAMASAKI, Sayaka, Suwa-shi, 392-8502 (JP)
(74) Representative: Lewis Silkin LLP

(57) **Abstract**

A cellulose saccharification method includes: a pulverization step of pulverizing a raw material containing cellulose and filler; a classification step of classifying the raw material in air into the cellulose and the filler; and a saccharification step of introducing the cellulose classified in the classification step and a saccharification reaction liquid which contains an enzyme in a saccharification tank to proceed a saccharification reaction by decomposition of the cellulose using the enzyme.

## Description

The present application is based on, and claims priority from JP Application Serial Number 2022-019357, filed February 10, 2022, the disclosure of which is hereby incorporated by reference herein in its entirety.

### BACKGROUND

### 1. Technical Field

The present disclosure relates to a cellulose saccharification method.

### 2. Related Art

In view of so-called carbon minus, effective use of biomass, application to bioethanol, and the like, attention has been paid on formation of glucose by cellulose saccharification. For example, JP-T-2014-507148 has disclosed a method for producing saccharides by saccharification using paper as a raw material. In the technique described in JP-T-2014-507148, a method for producing saccharides using waste paper as a cellulose raw material through a pre-treatment and an enzymatic saccharification has been disclosed.

When cellulose is saccharified, there has been a method in which after a raw material and a saccharification reaction liquid containing an enzyme are charged in a saccharification tank, an enzymatic reaction to saccharify the cellulose is performed by stirring. However, when paper is used as the raw material for cellulose, besides the cellulose, filler, such as CaCO₃, may be contained in the paper in some cases. When CaCOs is mixed in the saccharification reaction liquid, foaming may occur in some cases, and as a result, the saccharification reaction liquid overflows, and/or pipes are clogged in some cases.

Hence, a cellulose saccharification method which is not likely to generate foaming of a saccharification reaction liquid caused by filler contained in a raw material has been desired.

### SUMMARY

According to an aspect of the present disclosure, there is provided a cellulose saccharification method comprising: a pulverization step of pulverizing a raw material containing cellulose and filler; a classification step of classifying the raw material in air into the cellulose and the filler; and a saccharification step of introducing the cellulose classified in the classification step and a saccharification reaction liquid which contains an enzyme in a saccharification tank to proceed a saccharification reaction by decomposition of the cellulose using the enzyme.

### BRIEF DESCRIPTION OF THE DRAWINGS

FIGURE is a schematic view showing a saccharification treatment according to an example.

### DESCRIPTION OF EXEMPLARY EMBODIMENTS

Hereinafter, embodiments of the present disclosure will be described. The following embodiments are described to explain examples of the present disclosure. The present disclosure is not at all limited to the following embodiments and includes various modified embodiments to be performed without departing from the scope thereof. In addition, all the structures described bellow are not always required to be essential structures of the present disclosure.

A cellulose saccharification method according to this embodiment includes a pulverization step of pulverizing a raw material containing cellulose and filler; a classification step of classifying the raw material in air into the cellulose and the filler; and a saccharification step of introducing the cellulose classified in the classification step and a saccharification reaction liquid in a saccharification tank to proceed a saccharification reaction.

### 1. PULVERIZATION STEP

The pulverization step pulverizes a raw material containing cellulose and filler.

### 1.1. RAW MATERIAL

The raw material contains cellulose and filler. The raw material may also contain components other than the cellulose and the filler. As the components other than the cellulose and the filler, for example, a lignin and a hemicellulose may be mentioned as components derived from wood, and a pigment, a resin component, a clay, a binder, a toner, an oil component, moisture, and the like may be mentioned as components derived from processed wood.

The raw material may also be paper. When paper is used as the raw material, although containing cellulose and filler, the raw material can be easily obtained. In addition, the paper may also include printed waste paper. As the printed waste paper, for example, copy paper, newspapers, and magazines may be mentioned. When the printed waste paper is used, for example, environmental resources, underground resources, and the like are saved, and the waste can be preferably reduced.

The raw material is more preferably used after being sterilized. As a sterilizing method, for example, heating or ultraviolet radiation may be mentioned. Accordingly, for example, glucose produced by a saccharification reaction is not likely to be consumed by bacteria and the like derived from the raw material, and the yield of saccharides may be increased in some cases.

### 1.2. PULVERIZATION METHOD

Although a method for pulverizing the raw material is not particularly limited, for example, a cutting method by a shredder or the like or a pulverization (defibration) method by a dry defibration machine or the like may be used. Although the pulverization of the raw material is preferably performed by a dry operation, for example, wet defibration may also be performed.

When the raw material is in a pulverized state, since components contained in the raw material other than the cellulose are likely to be separated therefrom, and the cellulose is likely to be in contact with the reaction liquid, the efficiency of the saccharification step can be improved.

### 2. CLASSIFICATION STEP

In the classification step, fine particles in the raw material pulverized in the pulverization step are decreased. The classification step is performed in air. The classification step may be performed, for example, using a cyclone, a sieve, or the like. Since the throughput is more preferable, the classification step is more preferably performed using a cyclone.

An average particle diameter of the fine particles which are decreased in the classification step is 10 µm or less, preferably 5 µm or less, more preferably 3 µm or less, and further preferably 2 µm or less. In general, the particle diameter of the filler, such as calcium carbonate, is small. Hence, when the average particle diameter of the fine particles which are decreased in the classification step is in the range described above, the filler in the raw material can be decreased.

In the classification step, substances, such as a toner and a binder, in the raw material other than the cellulose may also be decreased. Accordingly, the saccharification reaction can be suppressed from being disturbed by the substances contained in the raw material which are not involved in the saccharification reaction.

In addition, in the case in which calcium carbonate is decreased in the classification step, when the raw material and the saccharification reaction liquid are mixed together, the pH of the mixture thus obtained can be suppressed from being increased. Furthermore, when calcium carbonate is decreased in the classification step, a neutralization reaction between an acid and an alkali in the saccharification reaction liquid can also be suppressed, and hence, the foaming can also be suppressed. As a result, for example, the clogging of pipes and/or the overflow of the mixture from the saccharification tank, each of which is caused by the foaming, can be suppressed.

### 3. SACCHARIFICATION STEP

In this step, after the raw material containing cellulose and filler and the saccharification reaction liquid are introduced in the saccharification tank, stirring is performed at an appropriate pH and temperature to saccharify the cellulose.

### 3.1. SACCHARIFICATION TANK

The saccharification tank is not particularly limited as long as the raw material and the reaction liquid can be introduced therein and can also be stirred. The scale of the saccharification tank is also not limited, and an experimental laboratory scale, such as a beaker or a flack, a pilot plant scale, or a commercial plant scale may be appropriately selected.

The saccharification tank may also include a container and a lid. The saccharification tank may also appropriately include, for example, inlets of the raw material and the reaction liquid, an outlet of the product, a mechanism to stir the inside, a window for internal observation, a heating heater, a refrigerant pipe for cooling, a jacket, and other pipes. Furthermore, the saccharification tank may also include a liquid level meter, a thermometer, and the like and may further have openings provided for the meters mentioned above.

As the stirring mechanism, for example, a magnetic stirrer and a stir bar; and a stirring motor, a shaft, and a stirring blade may be mentioned and may be appropriately selected in accordance with the scale and the stirring efficiency of the mixture.

### 3.2. SACCHARIFICATION REACTION LIQUID

The saccharification reaction liquid contains an enzyme, and water is also contained as a primary component. In the saccharification reaction liquid, besides the enzyme and the water, for example, substances, such as a pH adjuster and/or a surfactant, useful for the enzymatic reaction may also be contained.

### 3.2.1. Enzyme

As the enzyme, any enzyme which decomposes cellulose into saccharides by cleaving β-1,4-glucocide bonds may be used. As an example of the cellulolytic enzyme, for example, endoglucanase, cellobiohydrolase, or cellobiase (β-glucosidase) may be mentioned. As a more concrete example of the cellulolytic enzyme, for example, Cellulase SS (manufactured by Nagase ChemteX Corporation), Accellerase Duet (manufactured by GENENCOR), Cellic Ctec2 (manufactured by Novozymes), Cellic Ctec3 (manufactured by Novozymes), or Meicelase (manufactured by Meiji Seika Pharma Co., Ltd.) may be mentioned. At least two types of those enzymes may be used in combination. In addition, in order to improve the saccharification efficiency by simultaneously decomposing xylans present on the cellulose surface, a xylanase may also be contained.

An enzyme in the form of a powder, a solution, or a dispersion may be mixed in the saccharification reaction liquid. In addition, the enzyme may be mixed in advance in the saccharification reaction liquid or may be added to the saccharification reaction liquid in the saccharification tank.

### 3.2.2. Water

As the water, for example, pure water, such as ion exchange water, ultrafiltration water, reverse osmosis water, or distilled water, or water, such as ultrapure water, in which ionic impurities are removed as much as possible is preferable. In addition, in the case in which water sterilized by ultraviolet radiation, addition of hydrogen peroxide, or the like is used, when the reaction liquid is stored for a long time, generation of fungi and bacteria can be preferably prevented during and after the enzymatic reaction.

The saccharification reaction liquid may also contain a pH adjuster. As the pH adjuster, for example, there may be mentioned at least one selected from an organic acid, such as acetic acid or citric acid, an inorganic acid such as phosphoric acid, an organic alkali, an inorganic alkali, and salts, such as a sodium salt, of those mentioned above, or a substance forming a buffer solution.

The saccharification reaction liquid may also contain a surfactant. Any surfactant may be used as long as not disturbing the enzymatic reaction. When the surfactant is contained in the saccharification reaction liquid, the saccharification reaction liquid is likely to wet the raw material, and the efficiency of the saccharification reaction can be improved. In the saccharification method according to this embodiment, since the raw material is pulverized, and the surface area thereof is increased thereby, the raw material and the saccharification reaction liquid are not likely to wet each other; hence, the effect of improving the wettability by the use of the surfactant is more significant.

When the surfactant is used, a surfactant having a defoaming effect is more preferably contained. The surfactant having a defoaming effect may also be called a defoaming agent in some cases. Although the defoaming agent is not particularly limited, for example, there may be mentioned a silicone-based defoaming agent, a polysiloxane-based defoaming agent, an acetylene glycol-based defoaming agent, a polyether-based defoaming agent, or an aliphatic acid ester-based defoaming agent.

As a commercially available product of the silicone-based defoaming agent, for example, a three-dimensional siloxane "FOAM BAM (registered trademark) MS-575" (trade name, manufactured by Munzing), KM-71 or KM-75 (trade name, manufactured by Shin-Etsu Chemical Co., Ltd.), or BYK-093 or BYK-094 (trade name, manufactured by BYK Japan KK) may be mentioned.

As a commercially available product of the polysiloxane-based defoaming agent, for example, there may be mentioned KM-73A, KM-73E, KM-71, KM-85, KM-89, KM-98, KM-7752, KS-531, KS-540, KS-530, KS-537, or KS-538 (trade name, manufactured by Shin-Etsu Chemical Co., Ltd.), BYK-020, BYK-021, BYK-022, BYK-023, BYK-024, BYK-044, or BYK-094 (trade name, manufactured by BYK Japan KK), or TSA6406, TSA780, TSA739, or TSA775 (trade name, manufactured by Momentive Performance Materials Japan LLC.).

As the acetylene glycol-based defoaming agent, for example, there may be mentioned 2,4,7,9-tetramethyl-5-decyne-4,7-diol, an alkylene oxide adduct thereof, 2,4-dimethyl-5-decyne-4-ol, or an alkylene oxide adduct thereof. In addition, as a commercially available product of the acetylene glycol-based defoaming agent, for example, there may be mentioned Olfine 104 series or E series, such as Olfine E1010 (trade name, manufactured by Air Products Japan, Inc.) or Surfynol 465, 61, or DF110D (trade name, manufactured by Nisshin Chemical Industry Co., Ltd.).

When the saccharification reaction liquid contains a defoaming agent, since foaming is not likely to be generated, and even if being generated, the foaming can be easily stopped, for example, the overflow from the saccharification tank and/or the clogging of pipes can be further suppressed.

The saccharification reaction liquid may also contain a surfactant other than the defoaming agents mentioned above. Although the surfactant as described above is not limited to the following surfactants, for example, there may be mentioned a silicone-based, a polyoxyethylene alkyl ether-based, a polyoxypropylene alkyl ether-based, a polycyclic phenyl ether-based, a sorbitan derivative-based, a fluorine-based, or a nonionic-based surfactant.

### 3.3. pH

The pH of the saccharification reaction liquid in the saccharification step is adjusted in accordance with an optimum pH of an enzyme to be used. For example, when the enzyme to be used is Cellic Ctec2 (manufactured by Novozymes), the pH of the saccharification reaction liquid is 4.5 to 6.0 and is preferably 5.0 to 5.7. The adjustment of the pH may be performed by addition of the pH adjuster described above. In addition, the adjustment of the pH is more preferably performed before the addition of the enzyme. Furthermore, when the saccharification reaction liquid is mixed with the raw material, the pH of the saccharification reaction liquid may be increased in some cases. In the case as described above, the pH of the saccharification reaction liquid is preferably adjusted in the range described above.

The pH may be adjusted by addition of an aqueous solution of sodium acetate, acetic acid, sulfuric acid, sodium hydroxide, or the like to the saccharification reaction liquid. In addition, when the saccharification reaction liquid is configured such that the pH thereof is monitored, the pH may be adjusted in the saccharification step. For example, when the pH is increased to more than a predetermined pH while the stirring is performed in the saccharification tank, acetic acid, sulfuric acid, or the like is added, and when the pH described above is decreased to less than a predetermined pH, a sodium hydroxide aqueous solution or the like is added.

### 3.4. TEMPERATURE

A liquid temperature of the saccharification reaction liquid in the saccharification step is preferably adjusted to an optimum temperature of the enzyme to be used. For example, when the enzyme to be used is Cellic Ctec2 (manufactured by Novozymes), the temperature of the saccharification reaction liquid is 47°C to 52°C, preferably 48°C to 51°C, and more preferably 49°C to 50°C.

The temperature of the liquid in the saccharification tank is adjusted using an appropriate heating device, cooling device, controlling device, and/or the like.

### 3.5. STIRRING

In the saccharification step, the raw material and the saccharification reaction liquid described above are introduced in the saccharification tank and are then stirred, so that saccharides derived from the cellulose are produced. Although depending on the ability of the enzyme and the entire scale, the saccharification step is performed for a period of 2 hours to 1 week. The period in which the saccharification step is performed is typically 10 hours to 5 days, more preferably 1 to 4 days, and further preferably 2 to 3 days.

The stirring in the saccharification step is performed by the appropriate stirring mechanism described above. When the stirring is performed, for example, the rotation rate of a motor can be appropriately set in accordance with the scale and the structure of the saccharification tank, the shape of the stirring bar or the stirring blade, and the like.

### 4. OTHER STEPS

The saccharification method according to this embodiment may further include, besides the steps described above, a recovery step, a washing step, and the like.

### 4.1. RECOVERY STEP

The saccharification method may also include, after the saccharification step, a step of recovering the liquid in the saccharification tank. The recovery step may be performed through a pipe or the like. The liquid containing saccharides thus recovered may be processed by a filtration treatment.

### 4.2. WASHING OF SACCHARIFICATION TANK

The saccharification method may also include, after the saccharification step, a step of washing the saccharification tank. The step of washing the saccharification tank may be performed, for example, before and/or after the recovery step and may be a step of removing precipitates remaining in the saccharification tank, a step of washing an oil component remaining in the saccharification tank, a step of washing the stirring mechanism, and/or the like.

### 5. OPERATIONAL EFFECT

In the saccharification method of this embodiment, as a pre-treatment to be performed before the saccharification step, since the raw material containing cellulose is pulverized and then further classified, the filler contained in the raw material is decreased. Accordingly, since the saccharification step can be performed while the content of impurities is small, the saccharification reaction efficiency can be improved. In addition, when paper is used as the raw material, a basic component, such as CaCO₃, used as the filler may be contained in some cases, and as a result, foaming may be generated in some cases by a neutralization reaction and the like. Accordingly, for example, the overflow of the saccharification reaction liquid from the saccharification tank and/or the clogging of pipes may occur in some cases. However, by the saccharification method of this embodiment, since the filler is decreased by the classification, for example, the overflow of the saccharification reaction liquid from the saccharification tank and/or the clogging of pipes can be suppressed.

### 6. CONCRETE EXAMPLE of STRUCTURE

Hereinafter, although the present disclosure will be described in more detail with reference to concrete examples and the like, the present disclosure is not limited to the following examples.

### 6.1. STRUCTURE

FIGURE is a schematic view showing one example of a structure of capable of performing the saccharification method of the present disclosure. The structure of this example includes a coarsely pulverizing portion 10, a defibrating portion 20, a classification portion 30, and a saccharification tank 100, and the raw material is introduced in the saccharification tank 100 through the coarsely pulverizing portion 10, the defibrating portion 20, and the classification portion 30.

### 6.1.1. EXAMPLE OF SACCHARIFICATION TANK

As shown in FIGURE, in the saccharification tank 100, a mixture L of the saccharification reaction liquid and the raw material is introduced. A stirring shaft 20 and a stirring blade 10 are rotated by a motor (not shown), and the mixture L is stirred thereby. By the stirring, the cellulose is saccharified in the mixture L by an enzymatic reaction.

### 6.1.2. EXAMPLE OF COARSELY PULVERIZING PORTION

In the apparatus shown in FIGURE, the coarsely pulverizing portion 10 is disposed upstream of the defibrating portion 20. The coarsely pulverizing portion 10 cuts the raw material, such as waste paper, in air. The shape and the size of the raw material thus cut are not particularly limited, and for example, the size thereof is approximately several centimeters square. In the example shown in FIGURE, the coarsely pulverizing portion 10 has coarsely pulverizing blades 11, and the raw material thus introduced can be cut by the coarsely pulverizing blades 11. In the coarsely pulverizing portion 10, an automatic charging portion (not shown) to continuously charge the raw material may also be provided.

As a concrete example of the coarsely pulverizing portion 10, for example, a shredder may be mentioned. In the example shown in FIGURE, the raw material cut by the coarsely pulverizing portion 10 is received by a hopper 15 and is then transported to the defibrating portion 20 through a pipe 41. The pipe 41 communicates with an inlet 21 of the defibrating portion 20.

### 6.1.3. EXAMPLE OF DEFIBRATING PORTION

The defibrating portion 20 is provided upstream of the classification portion 30 which will be described later. The defibrating portion 20 performs a defibrating treatment of the coarsely pulverized raw material. The defibrating portion 20 performs dry defibration in the air (in air). In addition, the defibration is one type of pulverization. In the example shown in FIGURE, the raw material introduced from the inlet 21 is defibrated by the defibrating portion 20 into a defibrated material (pulverized material) and is then discharged from an outlet 22 so as to be supplied to the classification portion 30 through a pipe 42.

In addition, in this specification, the dry operation indicates an operation to be performed not in a liquid but in the air (in air). The dry operation includes an operation to be performed under a dry condition and an operation to be performed under a condition in which a liquid (such as water) is present as an impurity or under a condition in which a liquid (such as water), steam, mist, or the like is present by intentional addition. In addition, it should be noted that between the dry operation and a wet operation to be performed for paper making or the like, an amount of water to be used with respect to the entire apparatus or to the amount of paper to be manufactured is extremely different. That is, in the dry operation, the amount of water present in the system is significantly smaller than that in the wet operation by the order of magnitude.

Although the structure of the defibrating portion 20 is not particularly limited, for example, there may be mentioned a structure which includes a rotational portion (rotor) and a fixing portion to cover the rotational portion and in which a space (gap) is formed between the rotational portion and the fixing portion.

### 6.1.4. EXAMPLE OF CLASSIFICATION PORTION

In the apparatus shown in FIGURE, the classification portion 30 is disposed downstream of the defibrating portion 20. The classification portion 30 separates and removes fine particles of calcium carbonate (filler), resin particles, ink particles, and the like from the defibrated material. As the classification portion 30, an air stream classifier is preferably used. The air stream classifier generates a swirling airflow, and particles are classified in accordance with the sizes and the densities thereof using a centrifugal force. By adjustment of the velocity of the air stream and the centrifugal force, the classification point can be adjusted. In particular, as the classification portion 30, for example, a cyclone, an elbow-jet classifier, or an Eddy classifier may be used. In particular, since having a simple structure, the cyclone can be preferably used as the classification portion 30. Hereinafter, the case in which a cyclone is used as the classification portion 30 will be described.

The classification portion 30 includes an inlet 34, a cylindrical portion 35 connected to the inlet 34, an inverted corn portion 36 which is located under the cylindrical portion 35 and is connected thereto, a lower side outlet 37 provided at a bottom center of the inverted corn portion 36, and an upper side outlet 38 provided at a top center of the cylindrical portion 35.

In the classification portion 30, a motion of an air stream conveying the defibrated material introduced from the inlet 34 is changed to a circular motion in the cylindrical portion 35. Accordingly, the centrifugal force is applied to the defibrated material thus introduced, and as a result, the defibrated material can be separated into fibers and fine particles, such as calcium carbonate (filler), resin particles, and ink particles. Fiber-rich components are discharged from the lower side outlet 37 and are then introduced in the saccharification tank 100 through a tube 46. On the other hand, the fine particles are discharged outside of the classification portion 30 from the upper side outlet 38 through a pipe 44. In the example shown in FIGURE, the pipe 44 is connected to a receiving portion 39, and the fine particles are recovered in the receiving portion 39.

In addition, although it was described that the fibers and the fine particles are separated from each other by the classification portion 30, the fibers and the fine particles cannot be ideally separated from each other. For example, among the fibers, fibers having a relatively small size and/or fibers having a relatively low density may be discharged outside together with the fine particles in some cases. In addition, among the fine particles, particles having a relatively high density and/or particles entangled with the fibers may be discharged downstream together with the fibers in some cases.

### 6.2. EXAMPLES

An effect of the pulverization and classification on the efficiency of the cellulose saccharification was confirmed by the following experiments. After one liter of a sodium acetate buffer solution and an enzyme, Cellic Ctec2 (manufactured by Novozymes), were charged in a 2-liter beaker, acetic acid was added to adjust the pH at 5.2 to form a mixture, and by the same procedure as described above, two sets of the mixture as described above were prepared. In one of the two sets, 50 g of pulverized waste paper was charged. In the other set, 50 g of waste paper was charged after pulverized and then processed using a cyclone to decrease fine particles having a size of 3 µm or less. In addition, the mixture of each of the two sets was controlled to have a temperature of 49°C, and a reaction was performed by stirring. The behavior of each mixture was videoed, and the height of a liquid surface in the beaker was observed.

According to the experiment results, when the fine particles were decreased, the liquid surface height was approximately 50% of the height of the beaker at an initial stage and was not increased to 60% or more of the height of the beaker during the reaction. On the other hand, when the fine particles were not decreased, although the liquid surface height was approximately 50% of the height of the beaker at an initial stage, the liquid surface height was increased to 90% of the height of the beaker during the reaction in some cases. From the results described above, it was found that according to the saccharification method of the present disclosure, the foaming of the saccharification reaction liquid is suppressed in the saccharification step, and the saccharification reaction can be stably carried out.

The embodiments and modified examples described above are examples of the present disclosure, and the present disclosure is not limited thereto. For example, the embodiments and the modified examples may be appropriately performed in combination.

The present disclosure includes substantially the same structure as the structure described in the embodiment. That is, the substantially the same structure includes, for example, the structure in which the function, the method, and the result are the same as those described above, or the structure in which the object and the effect are the same as those described above. In addition, the present disclosure includes the structure in which a nonessential portion of the structure described in the embodiment is replaced with something else. In addition, the present disclosure includes the structure which performs the same operational effect as that of the structure described in the embodiment or the structure which is able to achieve the same object as that of the structure described in the embodiment. In addition, the present disclosure includes the structure in which a known technique is added to the structure described in the embodiment.

From the embodiments and modified examples described above, the following conclusions can be obtained.

A cellulose saccharification method includes: a pulverization step of pulverizing a raw material containing cellulose and filler; a classification step of classifying the raw material in air into the cellulose and the filler; and a saccharification step of introducing the cellulose classified in the classification step and a saccharification reaction liquid which contains an enzyme in a saccharification tank to proceed a saccharification reaction by decomposition of the cellulose using the enzyme.

According to the cellulose saccharification method described above, as a pre-treatment to be performed before the saccharification step, when the raw material containing cellulose is pulverized and is further classified, the filler contained in the raw material can be decreased. Accordingly, since the saccharification step can be performed in the state in which the filler is decreased, the foaming of the saccharification reaction liquid can be suppressed in the saccharification step, and hence, the saccharification reaction can be stably carried out.

In the cellulose saccharification method described above, the raw material may include paper.

According to the cellulose saccharification method described above, the raw material can be easily obtained.

In the cellulose saccharification method described above, the saccharification reaction liquid may have a pH of 6.0 or less.

In the paper, a basic component, such as CaCO₃, may be contained as the filler in some cases. When this paper is charged in the saccharification tank as the raw material, since the saccharification reaction liquid is adjusted to be acidic, the foaming occurs by a neutralization reaction. As a result, the overflow of the saccharification reaction liquid from the saccharification tank and/or the clogging of pipes may occur in some cases. However, according to this cellulose saccharification method, the foaming is suppressed, and furthermore, the saccharification reaction can be efficiently performed.

In the cellulose saccharification method described above, the saccharification reaction liquid may contain a surfactant.

In general, since the pulverized cellulose has a low apparent specific gravity, when being simply introduced in the saccharification reaction liquid, the cellulose is not likely to be immersed therein. However, according to this cellulose saccharification method, since the saccharification reaction liquid contains a surfactant, the wettability of the cellulose is improved, and the reaction efficiency is also improved. In addition, according to this cellulose saccharification method, the foaming of the saccharification reaction liquid can be further suppressed.

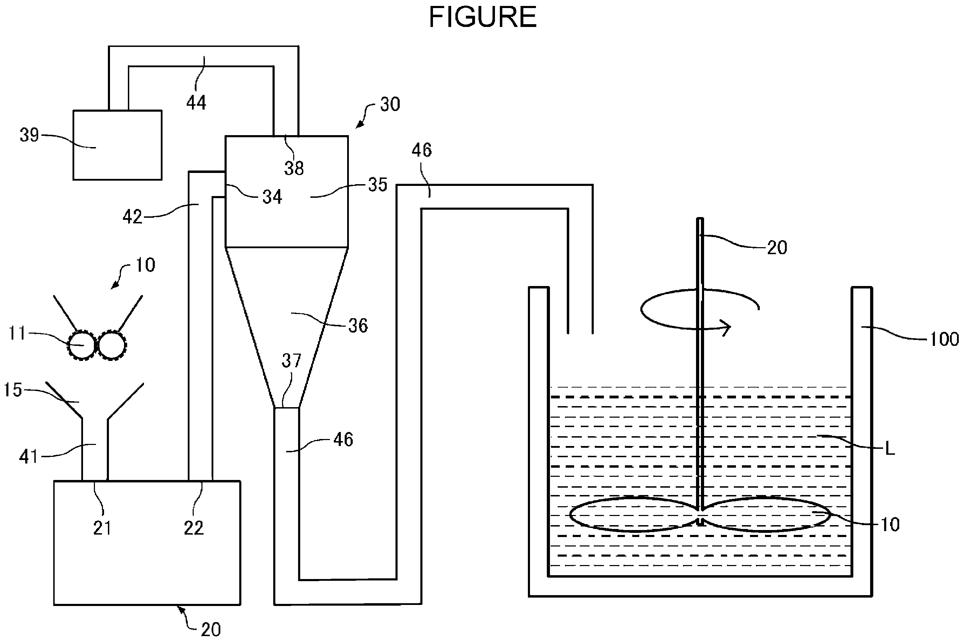

## Claims

1. A cellulose saccharification method comprising:
a pulverization step of pulverizing a raw material containing cellulose and filler;
a classification step of classifying the raw material in air into the cellulose and the filler; and
a saccharification step of introducing the cellulose classified in the classification step and a saccharification reaction liquid which contains an enzyme in a saccharification tank to proceed a saccharification reaction by decomposition of the cellulose using the enzyme.

2. The cellulose saccharification method according to claim 1,
wherein the raw material includes paper.

3. The cellulose saccharification method according to claim 1,
wherein the saccharification reaction liquid has a pH of 6.0 or less.

4. The cellulose saccharification method according to claim 1,
wherein the saccharification reaction liquid contains a surfactant.
